# EUROPÄISCHE PATENTSCHRIFT

(11) **EP 1 042 671 B1**
(45) Veröffentlichungstag und Bekanntmachung des Hinweises auf die Patenterteilung: **21.04.2004**
(21) Anmeldenummer: 98966367.9
(22) Anmeldetag: 18.12.1998
(51) Int. Cl.: G01N 33/53, C12Q 1/37

(54) **NACHWEIS VON ADENOSIN IN KLEINEN PROBEN**
ADENOSINE DETECTION IN SMALL SAMPLES
MISE EN EVIDENCE DE LA PRESENCE D'ADENOSINE DANS DE PETITS ECHANTILLONS

(30) Priorität: 23.12.1997 DE 19757571
(43) Veröffentlichungstag der Anmeldung: 11.10.2000
(73) Patentinhaber: Osswald, Hartmut, 72076 Tübingen (DE)
(72) Erfinder: OSSWALD, Hartmut, D-72076 Tübingen (DE); KLOOR, Doris, D-71032 Böblingen (DE)
(74) Vertreter: Tesch, Rudolf, Dr.
(86) Internationale Anmeldenummer: PCT/EP1998/008314
(87) Internationale Veröffentlichungsnummer: WO 1999/034210

(56) Entgegenhaltungen:
- BIOLOGICAL ABSTRACTS, Philadelphia PA USA; abstract no. PREV199800291792, siehe Zusammenfassung XP002103818 & D. KLOOR ET AL.: "A simple and sensitive assay for the measurement of adenosine in small samples." DRUG DEVELOPMENT RESEARCH, Bd. 43, Nr. 1, 1. Januar 1998, Seite 33 New York NY USA
- CHEMICAL ABSTRACTS, vol. 125, no. 19, 4. November 1996 Columbus, Ohio, US; abstract no. 241657, XP002103819 & D. KLOOR ET AL.: "S-adenosylhomocysteine-hydrolase from bovine kidney. Enzymatic and binding properties" KIDNEY BLOOD PRESSURE RESEARCH, Bd. 19, Nr. 2, 1996, Seiten 100-108, New York NY USA

## Beschreibung

Die Erfindung betrifft ein Nachweisverfahren zum Messen von Adenosin in kleinen Proben bei welchem zu einer auf Adenosin zu untersuchenden Flüssigkeit reduzierte S-Adenosylhomocystein-Hydrolase sowie markiertes Adenosin, jeweils in definierten Mengen, zugegeben wird, danach der S-Adenosylhomocystein-Hydrolase-Adenosin-Komplex, vorzugsweise mit einer Filtrationsapparatur abgetrennt wird und anschließend die in diesem Komplex vorhandene Markierung gemessen wird.

Ein weiterer Gegenstand der Erfindung ist eine Zusammenstellung der für den Test erforderlichen Analyse-Substanzen.

Adenosin ist ein endogenes Nukleosid, das als allgemein regulatorische Substanz zusammen mit anderen Hormonen und Neurotransmittern in physiologische Prozesse eingreift. Die Wirkungen von Adenosin werden über Rezeptoren an der Zelloberfläche vermittelt, die ubiquitär in Säugetieren zu finden sind.

Adenosin entsteht in der Zelle zum einen aus dem Abbau von AMP durch endo- und ekto-5'-Nucleotidasen, zum anderen durch die Hydrolyse von S-Adenosylhomocystein (SAH) zu Adenosin und Homocystein durch die SAH-Hydrolase. Als Abbauprodukt der energiereichen Phosphate hat Adenosin allgemein die Funktion des Mediators der metabolischen Kontrolle der Organfunktion in Herz, Niere und ZNS. In der biomedizinischen Forschung (Biochemie, Pharmakologie, Physiologie, klinische Chemie) nimmt das Interesse des Nachweises von endogenem Adenosin u.a. deswegen zu, weil in der letzten Zeit Adenosin-Rezeptor antagonisierende Substanzen als Arzneimittel in die Therapie eingeführt werden. Im Rahmen dieses gestiegenen Interesses am Adenosin nimmt auch die Forschung in den Laboratorien als auch in den Universitätskliniken weltweit zu um zu belegen, daß unter verschiedenen Funktionszuständen die Adenosinkonzentrationen in den Körperflüssigkeiten geändert werden.

Zur Messung von Adenosin stehen gegenwärtig mehrere Methoden zur Verfügung:

### 1. Spektralphotometer

Am Photometer wird der Abbau von Adenosin durch die Adenosindesaminase bei einer Wellenlänge von 265 nm gemessen. Der Nachteil dieser Meßmethode besteht darin, daß die zu messende Probe frei sein muß von interferierenden Substanzen, was zu einer zusätzlichen Aufreinigung der Probe führt. Außerdem liegt die Nachweisgrenze für Adenosin im Photometer bei 10⁻⁷ M.

### 2. Hochdruckflüssigkeitschromatographie (HPLC)

Auch die HPLC detektiert das Adenosin bei einer Wellenlänge von 265 nm. Eine Interferenz mit anderen Substanzen, bei dieser Wellenlänge, kann durch die geeignete Wahl des Fließmittels umgangen werden. Ein Nachteil dieser Methode ist die zusätzliche Aufreinigung der Proben. Hinzu kommt die geringe Sensitivität der HPLC, die ähnlich der des Photometers (3-10⁻⁷ M) ist.

### 3. Radioimmunoassay

Der RIA beruht auf einer Antikörper Methode mit Adenosin 2',3'-O-Disuccinyl-3-[¹²⁵I]-Iodtyrosin Methyl Ester als Tracer und einem Antikörper gegen Disuccinyliertes-Adenosin. Ein Vorteil dieser Methode liegt darin, daß eine Aufreinigung der Proben entfällt, nachteilig ist die Succinylierung jeder Probe vor der Messung. Die Nachweisgrenze dieses Tests liegt bei 6·10⁻⁹ M. Damit ist der RIA zum Nachweis von Adenosin empfindlicher als HPLC und Photometer. Ein weiterer Nachteil dieses Testes ist, daß er nur in Japan erhältlich und mit 1600.- DM für 40 Proben einschließlich Eichkurve recht teuer ist.

Aufgabe der Erfindung war es daher, ein Nachweisverfahren zu schaffen, das es ermöglicht, auch in kleinsten Proben von z.B. 10 µl, oder in Biopsien von 2-10 mg Gewebe, Adenosin messen zu können, das keine aufwendige Aufreinigung der Probe erfordert und sehr sensitiv ist.

Diese Aufgabe wird erfindungsgemäß dadurch gelöst, daß ein Nachweisverfahren zum Messen von Adenosin in kleinen Proben zur Verfügung gestellt wird, bei welchem zu einer auf Adenosin zu untersuchenden Flüssigkeit reduzierte S-Adenosylhomocystein-Hydrolase sowie markiertes Adenosin, jeweils in definierten Mengen, zugegeben wird, danach der S-Adenosylhomocystein-Hydrolase-Adenosin-Komplex, vorzugsweise mit einer Filtrationsapparatur abgetrennt wird und anschließend die in diesem Komplex vorhandene Markierung gemessen wird.

Dieses Nachweisverfahren beruht auf der Fähigkeit des Enzyms SAH-Hydrolase (reduzierte Form des Enzyms) Adenosin zu binden.

In der nachfolgenden Tabelle 1 sind die Werte aufgeführt die belegen, daß das reduzierte Enzym Adenosin bindet nicht aber die Substanzen die an das nicht reduzierte Enzym binden.

**Tabelle 1**

| **Verdrängung des 3H-Adenosins von der reduzierten und nicht reduzierten Form der SAH-Hydrolase durch Adenosin und Adenosin Analoga. Angegeben sind die EC**_{**50**}**-Werte in nmol/1.** | | |
|---|---|---|
| Adenosin Analoga | nicht-reduzierte SAH-Hydrolase | reduzierte SAH-Hydrolase |
| Adenosin | 57 | 24 |
| cAMP | 104 | > 100000 |
| Adenin | 11500 | > 100000 |
| 2'Desoxyadenosin | 94 | 630 |
| SAH | 246 | 400 |
| Homocystein | 5000 | 5000 |
| AMP | 10000 | 100000 |
| ADP | 10000 | > 100000 |
| AMP | 10000 | > 100000 |
| Inosin | 10000 | 10000 |
| Diadenosin-Diphosphat | 138 | 10000 |
| Diadenosin-Dialdehyd | 180 | 10000 |
| NECA | 240 | > 100000 |
| Theophyllin | > 100000 | > 100000 |

Die Vorteile dieses neuen Nachweisverfahrens bestehen darin, daß dieses enzymatisch inaktive Enzym weiterhin seine Fähigkeit Adenosin mit hoher Aktivität (K_{d} 20 nM) zu binden behält. Da dieses Enzym keine Interferenzen mit endogen vorkommenden Substanzen zeigt, entfällt eine weitere Aufreinigung der zu untersuchenden Proben.

Im folgenden soll die Erfindung an Hand eines möglichen Ausführungsbeispiels näher erläutert werden:

Gereinigt wird die SAH-Hydrolase aus Organen z.B. aus Rindernieren bis zur Homogenität.
Das Enzym wird mit klassischen chromatographischen Methoden gereinigt.
Die Rinderniere (600 g) wird mit 50 mM Kaliumphosphat Puffer pH 7.0, 1 mM DTT; 1 mM EDTA, im Verhältnis 1 Teil Niere, 2 Teile Puffer homogenisiert und anschließend für 45 Minuten bei 20000 x g und 4°C abzentrifugiert. Der Überstand wird mit einer 50% Ammoniumsulfatfällung (164 g/l) gefällt und durch Zentrifugation 30 Minuten bei 20000 x g wird das Präzipitat abgetrennt. Das Präzipitat wird in 300-400 ml 20 mM Tris/HCl Puffer pH 7.4, 1 mM DTT, 1 mM EDTA gelöst und über Nacht gegen den gleichen Puffer dialysiert.

### Säulenchromatographie

### 1. DEAE-Säule.

Die dialysierte Proteinlösung wird auf eine DEAE-Säule (25 x 5 cm) aufgetragen die gegen den 20 mM Tris/HCl Puffer pH 7.4, 1 mM DTT und 1 mM EDTA äquilibriert wurde. Nach Spülen der Säule mit weiteren 1000 ml des gleichen Puffers wird das Enzym entlang eines linearen Gradienten - 1000 ml Startpuffer und 1000 ml 500 mM KCl in oben genanntem Puffer - eluiert. Die SAH-Hydrolase eluiert bei einer Konzentration von 250-350 mM KCl (Laufgeschwindigkeit 10 ml/min). Die enzymatisch aktiven Fraktionen aus der Ionenaustauschchromatographie werden vereinigt (ca. 350 ml) und gegen Kaliumphosphatpuffer 10 mM pH 7.0 dialysiert.

### 2. Hydroxylapatit-Säulenchromatographie

Die dialysierte Enzymlösung wird anschließend über eine Hydroxylapatit Bio-Gel HTP Säule (10 x 5 cm) gegeben, welche vorher mit 10 mM Kaliumphosphatpuffer pH 7.0 äquilibriert wurde. Unter diesen Bedingungen wird die SAH-Hydrolase nicht an das Säulenmaterial gebunden, sondern erscheint in den Durchbruch-Fraktionen (Laufgeschwindigkeit 1.5 ml/min).

### 3. Aminohexyl Sepharose Chromatographie

Die enzymatisch aktiven Fraktionen werden vereinigt und auf eine 10 EAH-Sepharose 4B Säule 10 x 2.6) aufgetragen. Äquilibriert wird die Säule mit 10 mM Kaliumphosphatpuffer. Diese Säule dient neben der Aufreinigung der Aufkonzentrierung der Fraktionen von der Hydroxylapatit Säule. Das Enzym wird mit einem linearen 500 mM KCl Salzgradienten in 10 mM Kaliumphosphatpuffer pH 7.0 eluiert. Die enzymatisch aktiven Fraktionen, die bei einer Konzentration von 120 - 200 mM KCl eluierten, werden vereinigt und mit Hilfe eines Proteinkonzentrators (Ausschlußmolekulargewicht der Membran 30000 Dalton) auf 3 ml aufkonzentriert.

### 4. Superdex TM Gelfiltrations Chromatographie

Die konzentrierte Enzymlösung wird abschließend auf eine Hi Load TM 26/60 Superdex TM Säule (60 x 2.6 cm) aufgegeben, die mit PBS pH 7.4, 1 mM DTT und 1 mM EDTA äquilibriert wurde. Die Chromatographie wird mit dem gleichen Puffer bei einer Laufgeschwindigkeit von 0.2 ml/min durchgeführt. Die enzymatisch aktiven Fraktionen werden vereinigt und der Proteingehalt bestimmt.

Die Reinheit des isolierten Enzyms wird mit der SDS-Polyacrylamidgradientengel Elektrophorese überprüft.
Aus einer 600 g schweren Nieren werden 30 mg SAH-Hydrolase erhalten. Dieses Enzym wird durch Austausch des NAD⁺ mit NADH inaktiviert.

Die Inaktivierung des Enzyms kann mit drei unterschiedlichen Methoden durchgeführt werden:
1. Das enzymatisch aktive Enzym wird mit 150 mM NaCl, 8mM ATP und 8 mM MgCl₂ für 90 Minuten bei 37°C inkubiert. In dieser Zeit löst sich das fest gebundene NAD⁺ vom Enzym, das durch anschließende Dialyse entfernt wird. Es entsteht ein apo-Enzym ohne Enzymaktivität und ohne Adenosin-Bindungskapazität. mit einer 1 mM NADH Lösung wird das apo-Enzym für weitere 90 Minuten bei Raumtemperatur inkubiert. Danach wird das nicht gebundene NADH durch Dialyse entfernt und es resultiert ein enzymatisch inaktives Enzym mit hoher Adenosin-Bindungskapazität.
2. Das enzymatisch aktive Enzym wird in einem Verhältnis 1:2 mit gesättigter Ammoniumsulfatlösung 60 Minuten bei Raumtemperatur oder 0°C inkubiert. Nach 30 minütiger Zentrifugation bei 12000 x g wird das Pellet in 15 mM Tris/Hepes Puffer pH 7.0 gelöst und gegen den gleichen Puffer 6 Stunden mit 2 x Pufferwechsel dialysiert. Danach wird das apo-Enzym mit 1 mM NADH für 90 Minuten bei Raumtemperatur inkubiert. Das nicht gebundene NADH wird durch Dialyse entfertn. Die NADH-SAH-Hydrolase ist enzymatisch inaktiv, besitzt aber Adenosin Bindungskapazität.
3. Enzymatisch aktive SAH-Hydrolase wird mit 100 µM Azido-Adenosin für 2 Stunden bei Raumtemperatur inkubiert. Danach wird das Enzym-Azido-Adenosin-Gemisch 5 Minuten mit kurzwelligem UV-Licht (254 nm) bestrahlt. Dadurch bindet das Azido-Adenosin kovalent an die SAH-Hydrolase was zu einer enzymatischen Inaktivierung des Enzyms führt. Das überschüssige Azido-Adenosin wird durch Dialyse entfernt. Diese reduzierte SAH-Hydrolase ist enzymatisch inaktiv, bindet jedoch Adenosin mit hoher Kapazität.
   Bei der Reduktion der SAH-Hydrolase, unabhängig von der angewandten Methode, kommt es zu einem Verlust an Protein von 25% (entspricht 7.5 mg). Das inaktive Enzym (2 mg/ml) ist bei 4°C in Pufferlösung (20 mM Tris/Hepes pH 7.0) bis zu 4 Wochen stabil. Bei -20°C über 2 Monate. In lyophylisiertem Zustand (2 mg/ml) und bei -20°C ist dieses Enzym über 4 Monate lang stabil. Aus einer Rinderniere läßt sich soviel Bindungsprotein herstellen, daß etwa 20000 Proben auf ihre Adenosinkonzentration gemessen werden können.

### Nachweis von Adenosin durch Hemmungsexperiment:

Nachgewiesen wird das Adenosin mit Hilfe eines sogenannten Hemmungsexperiments. Dabei konkurriert das Adenosin aus der zu messenden Probe mit dem radioaktiv markierten Adenosin um die Bindungsstelle am reduzierten SAH-Hydrolase Molekül.

Zum Nachweis der Adenosin Konzentration werden pro Versuchsansatz, der ein Endvolumen von 300 µl enthält, folgende Einzelkomponenten zugegeben:
100 µl reduzierte SAH-Hydrolase (1 µg/300 µl)
50 µl ³H-Adenosin (1 pmol/300 µl) - Spezifische
Aktivität 62 Ci/mmol
100 µl Probe
50 µl Puffer - 20 mM Tris, 40 mM Hepes Puffer pH 7.4

Da die Assoziation von Adenosin an das reduzierte Enzym bei Raumtemperatur 10 Stunden dauert, werden die Proben über Nacht inkubiert. Das an das reduzierte Enzym gebundene radioaktiv markierte Adenosin wird von dem nicht gebundenen Adenosin über einen Nitrocellulosefilter mit einer Filtrationsapparatur getrennt. Dabei bleibt der Adenosin-Enzym-Komplex auf dem Filter hängen und die gebundene Radioaktivität wird mit einem Szintillationscounter gemessen

Damit das Adenosin aus der Probe ausgewertet werden kann, wird eine Eichkurve mit bekannten Adenosinkonzentrationen hergestellt. Eingesetzt werden Adenosin-Konzentrationen von 1 - 100 nM. Wenn nun der Logarithmus der eingesetzten Adenosin-Konzentrationen gegen die spezifische Bindung von ³H-Adenosin aufgetragen wird ergibt sich klassischerweise eine sigmoidale Dosis-Wirkungskurve. Der Anstieg dieser Kurve ergibt die Eichgerade. Anhand dieser Kurve läßt sich nun die unbekannte Adenosin-Konzentration in der Probe bestimmen.

Da die Affinität von Adenosin an das inaktive Enzym hoch ist und erfindungsgemäß ³H-Adenosin als Tracer benutzt wird, liegt die Nachweisgrenze für Adenosin in diesem Assay bei 10⁻¹⁰ M oder 10 fmole/Probe.

Dieses Nachweisverfahren kann für Forschungszwecke eingesetzt werden, wenn nur kleine Probenmengen zur Verfügung stehen und die Sensitivität der HPLC- oder anderen Methoden nicht ausreicht. Weitere Anwendungen des Radio-Enzym-Testes sind Kontrastmitteluntersuchungen und Nierentransplantationen als prognostische Aussage für die Nierenfunktion.

Die erfindungsgemäße Zusammenstellung von Analyse-Substanzen zur Bestimmung von Adenosin in kleinen Proben wird gemäß nachfolgendem Beispiel beschrieben.

Test-Kit zur Bestimmung von Adenosin enthaltend:
[2,8,5'-³H]-Adenosin 110 pmol (lyophylisiert)
Reduzierte SAH-Hydrolase 110 µg (lyophylisiert)
Adenosin 16.5 mg (pulv.)
Aktivkohle 2 g (pulv.)
Dextran 1 g (pulv.)
20 mM Tris/HCl Puffer ph 7.0 100 ml Konzentrat 2x (liquid.)

Zum erfindungsgemäßen Nachweis von Adenosin werden [2,8,5'-³H]-Adenosin und die reduzierte SAH-Hydrolase mit 20mM Tris/HCl Puffer ph 7.0 rekonstituiert. Aus den 16.5 mg Adenosin wird eine 6 mM Adenosin-Lösung hergestellt und entsprechend verdünnt, um eine Eichgerade mit Endkonzentrationen im Testansatz von 10, 25, 50, 75, 100, 150 und 200 nM zu haben.

Aktivkohle und Dextran werrden dazu benötigt, um eine 0.6% Aktivkohle und 0.2% Dextran enthaltende Lösung in 20 mM Tris/HCl pH 7.0 herzustellen.

Der Enzym-Ligand-Komplex muß mittels Filtration getrennt werden.

Durch Zugabe von 1 ml der Aktivkohle/Dextranlösung zum Testansatz und anschließende Zentrifugation kann der Enzym-Ligand-Komplex ebenfalls abgetrennt werden.

Zur analytischen Bestimmung werden jeweils 100 µl [2,8,5'-³H]-Adenosin, 100 µl SAH-Hydrolase und 100 µl Probe bzw. kaltes Adenosin für die Eichgerade eingesetzt.

## Patentansprüche

1. Verfahren zum Nachweis von Adenosin in Flüssigkeiten, **dadurch gekennzeichnet, daß** zu einer auf Adenosin zu untersuchenden Flüssigkeit reduzierte S-Adenosylhomocystein-Hydrolase sowie markiertes Adenosin, jeweils in definierten Mengen, zugegeben wird, danach der S-Adenosylhomocystein-Hydrolase-Adenosin-Komplex, vorzugsweise mit einer Filtrationsapparatur abgetrennt wird und anschließend die in diesem Komplex vorhandene Markierung gemessen wird.

2. Verfahren zum Nachweis von Adenosin in Flüssigkeiten nach Anspruch 1, **dadurch gekennzeichnet, daß** die Markierung eine radioaktive oder Fluoreszenzmarkierung ist.

3. Verfahren zum Nachweis von Adenosin in Flüssigkeiten nach Anspruch 2, **dadurch gekennzeichnet, daß** eine Probenvorbereitung und Aufreinigung, um störende andere Substanzen wie sie normalerweise in Körperflüssigkeiten gefunden werden zu entfernen, nicht mehr nötig ist.

4. Verfahren zum Nachweis von Adenosin in Flüssigkeiten nach Anspruch 1 bis 3, **dadurch gekennzeichnet, daß** Adenosin bei Kontrastmitteluntersuchungen gemessen wird.

5. Verfahren zum Nachweis von Adenosin in Flüssigkeiten nach Anspruch 1 bis 3, **dadurch gekennzeichnet, daß** Adenosin zur Bestimmung des Funktionszustandes isolierter Organe gemessen wird.

6. Zusammenstellung von Analyse-Substanzen zur Bestimmung von Adenosin in Flüssigkeiten, enthaltend [2,8,5'³H]-Adenosin, reduzierte SAH Hydrolase, Adenosin, Aktivkohle und Dextran.

7. Zusammenstellung von Analyse-Substanzen zur Bestimmung von Adenosin in Flüssigkeiten gemäß Anspruch 6, enthaltend 110 pm [2,8,5'³H]-Adenosin, 110 µg SAH Hydrolase, 16.5 mg Adenosin, 2 g Aktivkohle, 1 g Dextran und 100 ml (2x Konzentrat) 20 mM Tris/HCl pH 7.0.

## Claims

1. A method for the detection of adenosine in fluids, **characterized in that** to a sample of a fluid in which adenosine has to be determinated definite concentrations of reduced S-adenosylhomocysteine hydrolase and labeled adenosine are added, thereafter the S-adenosylhomocysteine hydrolase-adenosine-complex is separated, preferably with a filtration device, and subsequently the labelling incorporated in the enzyme-adenosine complex is measured.

2. A method for the detection of adenosine in fluids according to claim 1, **characterized in that** the labelling of adenosine is radioactive or fluorescent.

3. A method for the detection of adenosine in fluids according to claim 2, **characterized in that** a preliminary sample preparation and purifation for removing other interfering substances which are usually found in body fluids is not required.

4. A method for the detection of adenosine in fluids according to claims 1 to 3, **characterized in that** adenosine is measured during examinations with radio contrast media.

5. A method for the detection of adenosine in fluids according to claims 1 to 3, **characterized in that** adenosine is measured for the determination of the functional condition of isolated organs.

6. A kit of analytic substances for the determination of adenosine in fluids containing [2,8,5'³H]-adenosine, reduced SAH hydrolase, adenosine, activated charcoal and dextran.

7. The kit of analytic substances for the determination of adenosine in fluids according to claim 6 containing 110 pmol [2,8,5'³H]-adenosine, 110 µg reduced SAH hydrolase, 16.5 mg adenosine, 2 g activated charcoal, 1 g dextran and 100 ml (2.times.concentrate) 20 mM Tris/HCl pH 7.0.

## Revendications

1. Un procédé pour la détection d'adénosine dans les fluides, tel que un échantillon de fluide contenant de l'adénosine est mis en présence de S-adénosyl homocystéine hydrolase réduite et d'adénosine marqué, dans des quantités définies, par la suite le complexe S-adenosyl homocysteine hydrolase - adénosine est séparé, de préférence à l'aide d'un système de filtration, puis le marquage porté par le complexe enzyme-adénosine est mesuré.

2. Un procédé pour la détection d'adénosine dans les fluides selon la revendication 1, tel que le marqueur de l'adénosine est radioactif ou fluorescent.

3. Un procédé pour la détection d'adénosine dans les fluides selon la revendication 2, tel que la préparation préliminaire de l'échantillon et la purification en vue de l'élimination de substances interférentes qui sont usuellement présentes dans les fluides du corps ne sont plus nécessaires.

4. Un procédé pour la détection d'adénosine dans les fluides selon les revendications 1 à 3, tel que l'adénosine est mesurée par radiographie des contrastes.

5. Un procédé pour la détection d'adénosine dans les fluides selon les revendications 1 à 3, tel que l'adénosine est mesurée en vue de la détermination de l'état fonctionnel des organes isolés

6. Un kit de substances analytiques pour la détermination de l'adénosine dans les fluides contenant [2,8,5, ³H]-adénosine, SAH hydrolase réduite, adénosine, charcol actif et dextran.

7. Un kit de substances analytiques pour la détermination de l'adénosine dans les fluides selon la revendication 6 contenant 110 pm [2,8,5, ³H]-adénosine, 110 □g SAH hydrolase réduite, 16.5 mg adénosine, 2 g charcol actif, 1 g dextran et 100 ml (2 fois concentré) 20 mM Tris/HCl pH 7.0.
